# EUROPEAN PATENT SPECIFICATION

(11) **EP 4 244 183 B1**
(45) Date of publication and mention of the grant of the patent: **11.02.2026**
(21) Application number: 21806282.6
(22) Date of filing: 11.11.2021
(51) Int. Cl.: C01B 17/88, C07C 201/06, C07C 201/10

(54) **PROCESS FOR CONCENTRATING DILUTED SULFURIC ACID**
VERFAHREN ZUR KONZENTRIERUNG VERDÜNNTER SCHWEFELSÄURE
PROCÉDÉ DE CONCENTRATION D'ACIDE SULFURIQUE DILUÉ

(30) Priority: 11.11.2020 EP 20207013
(43) Date of publication of application: 20.09.2023
(73) Proprietor: BASF SE, 67056 Ludwigshafen am Rhein (DE)
(72) Inventor: KOELLER, Maximilian, 16244 Schorfheide (DE); BUETTNER, Johannes, 01986 Schwarzheide (DE); FRITZ, Ruediger, 02994 Bernsdorf (DE); MATTERN, Jonas, 01986 Schwarzheide (DE)
(74) Representative: BASF IP Association
(86) International application number: PCT/EP2021/081406
(87) International publication number: WO 2022/101353

(56) References cited:
- CA-A1- 2 236 620
- US-A- 4 157 381
- US-A- 4 772 757
- US-A1- 2016 083 332

## Description

The invention relates to a process for concentrating diluted sulfuric acid which may comprise at least one nitroaromatic compound and/or nitric acid as impurities by:
(a) feeding the diluted sulfuric acid into a first stage in which low boilers are removed by evaporation and/or stripping to obtain a first concentrated sulfuric acid;
(b) optionally feeding the first concentrated sulfuric acid into a second evaporation stage to obtain a second concentrated sulfuric acid;
(c) feeding the second concentrated sulfuric acid into a third evaporation stage if step (b) is carried out, or feeding the first concentrated sulfuric acid into the third evaporation stage if step (b) is not carried out, to obtain concentrated sulfuric acid as product.

Sulfuric acid which may comprise at least one nitroaromatic compound and/or nitric acid as impurities for example emanates from a process for producing nitroaromatic compounds by nitration of an aromatic compound by reaction with nitric acid and sulfuric acid.

Such nitroaromatic compounds particularly are nitrobenzene or nitrotoluene. In the process sulfuric acid is used as catalyst. After completing the reaction, the reaction mixture is separated into an organic phase comprising the nitroaromatic compound and an aqueous phase comprising diluted sulfuric acid. After separating the organic phase from the aqueous phase, the aqueous phase is concentrated to regain sulfuric acid which can be reused in the process for producing the nitroaromatic compound.

Usually the aqueous phase still contains remainders of the nitroaromatic compound produced in the process or organic byproducts and non-reacted nitric acid. These impurities are mainly removed in the first stage. Nevertheless, the sulfuric acid still contains small proportions of organic impurities in in the following evaporation stages, which leads to the formation of solid carbonaceous precipitates, particularly at higher temperatures. As the formation of solid precipitates causes equipment fouling and thereby impairs the plant performance, a plant shutdown for cleaning purposes can be necessary. In case of local organic accumulations, particularly in dead band areas at higher temperatures, there is a risk of spontaneous decomposition of the organic impurities which may be followed by pressure shocks and equipment damage.

WO-A 2014/177450 describes a process for producing nitrobenzene by adiabatic nitration of benzene with nitric acid and sulfuric acid, where the diluted sulfuric acid, which is separated as aqueous phase from the organic phase comprising the nitrobenzene, is concentrated by distillation. After concentrating, an oxidant is added to the sulfuric acid at least one minute before bringing the sulfuric acid into contact again with fresh nitric acid. However, by this process formation of solid precipitates and a spontaneous decomposition of organic impurities cannot be avoided.

Further processes for concentrating diluted sulfuric acid are described in US 4,157,381 or CA-A 2,236,620.

Object of the present invention is to provide a process for concentrating diluted sulfuric acid in which the formation of solid carbonaceous precipitates and the spontaneous decomposition of organic impurities are avoided and thus no fouling, pressure shocks and equipment damage occur.

This object is achieved by a process for concentrating diluted sulfuric acid which may comprise at least one nitroaromatic compound and/or nitric acid as impurities, comprising:
(a) feeding the diluted sulfuric acid into a first stage in which low boilers are removed by evaporation and/or stripping to obtain a first concentrated sulfuric acid;
(b) optionally feeding the first concentrated sulfuric acid into a second evaporation stage to obtain a second concentrated sulfuric acid;
(c) feeding the second concentrated sulfuric acid into a third evaporation stage if step (b) is carried out, or feeding the first concentrated sulfuric acid into the third evaporation stage if step (b) is not carried out, to obtain concentrated sulfuric acid as product;
wherein the third evaporation stage is operated at a pressure in a range from 10 to 800 mbar(abs) and a temperature in a range from 180 to 250 °C and wherein an oxidizing agent and/or a precursor of an oxidizing agent is fed into the third evaporation stage.

Surprisingly it has been shown that by feeding the oxidizing agent and/or the precursor of the oxidizing agent into the third evaporation stage which usually is operated at a temperature at which the formation of solid carbonaceous precipitates and a decomposition of the nitroaromatic compounds are initiated, both, fouling due to solid precipitates and spontaneous decomposition of the nitroaromatic compound which may result in pressure shocks and equipment damage, can be avoided. Further, by addition of the oxidizing agent and/or the precursor of the oxidizing agent into the third evaporation stage, also the total organic carbon (TOC) can be minimized.

According to the invention, feeding of the oxidizing agent and/or the precursor of the oxidizing agent into the third evaporation stage can be carried out for example by adding the oxidizing agent and/or the precursor of the oxidizing agent into the second concentrated sulfuric acid, if step (b) is carried out, or into the first concentrated sulfuric acid, if step (b) is not carried out, before the second concentrated sulfuric acid or the first concentrated sulfuric acid, respectively, is fed into the third evaporation stage. Additionally or alternatively, it is also possible to feed the oxidizing agent and/or the precursor of the oxidizing agent directly into the third evaporation stage, either into an apparatus of the third evaporation stage or into a loop through which the liquid which is evaporated is circulated.

The diluted sulfuric acid which may comprise at least one nitroaromatic compound and/or nitric acid for example emanates from a process for producing the nitroaromatic compound. Such processes for example are processes for producing nitrobenzene by nitration of benzene with nitric acid and sulfuric acid or for producing nitrotoluene by nitration of toluene with nitric acid and sulfuric acid. Particularly preferably, the inventive process is used for concentrating diluted sulfuric acid which contains nitrotoluene as impurities. If the diluted sulfuric acid contains nitrotoluene as organic impurities, the nitroaromatic compound particularly is at least one of mononitrotoluene, dinitrotoluene and/or dinitrocresols. If the diluted sulfuric acid emanates from a process for producing the nitroaromatic compound, it is particularly preferred to recycle the concentrated sulfuric acid which is obtained by the inventive process into the process for producing the nitroaromatic compound, particularly into a process for producing nitrotoluene.

The total amount of nitroaromatic compounds in the diluted sulfuric acid preferably is in a range from 0.01 to 10.0 wt%, more preferred in a range from 0.1 to 5.0 wt% and particularly in a range from 0.3 to 3.0 wt%, each based on the total amount of the diluted sulfuric acid.

If the diluted sulfuric acid also contains nitric acid, the concentration of nitric acid usually is in a range from 0.0 to 10.0 wt%, more preferred in a range from 0.1 to 5.0 wt% and particularly in a range from 0.4 to 3.0 wt%, each based on the total amount of the diluted sulfuric acid. Besides the nitroaromatic compounds and the nitric acid, the diluted sulfuric acid may contain further impurities, for example nitrous acid. The amount of further impurities may be in a range from 0.0 to 5.0 wt%, more preferred in a range from 0.0 to 2.0 wt% and particularly in a range from 0.1 to 1.0 wt%, each based on the total amount of the diluted sulfuric acid.

The diluted sulfuric acid may have a concentration in a range from 60 to 85 wt%, more preferred in a range from 65 to 79 wt% and particularly in a range from 70 to 75 wt%.

The concentrated sulfuric acid which is obtained as product from the third evaporation step preferably has a concentration in the range from 80 to 99 wt%, more preferred in a range from 85 to 97 wt% and particularly in a range from 90 to 95 wt%.

The concentration of the diluted sulfuric acid and the concentrated sulfuric acid, respectively, thereby is defined as amount of H₂SO₄ based on the amount of H₂SO₄ and water. Impurities in the diluted sulfuric acid are not considered for determining the concentration.

For concentrating, the diluted sulfuric acid is fed into the first stage in step (a). In the first stage, low boilers are removed from the diluted sulfuric acid. The low boilers which are removed in the first stage for example may comprise nitric acid, and major parts of organic components which are contained in the diluted sulfuric acid. Organic compounds which are removed from the diluted sulfuric acid in the first stage for example are mononitrotoluene isomers and dinitrotoluene isomers.

The first stage preferably comprises a simple evaporation, a stripping and/or a combination of evaporation and stripping. If the low boilers are removed by evaporation, the diluted sulfuric acid undergoes a temperature increase and the low boilers evaporate and can be removed in gaseous form. To evaporate the low boilers, the diluted sulfuric acid preferably is fed into an apparatus for evaporation and/or an apparatus for evaporation and stripping with a temperature in the range from 10 to 150 °C, more preferred with a temperature in the range from 15 to 120 °C and particularly in a range from 30 to 100 °C. In the apparatus for evaporation and/or the apparatus for evaporation and stripping the temperature preferably is in a range from 130 to 200 °C, more preferred in a range from 150 to 200 °C and particularly in a range from 150 to 190 °C. The pressure inside the apparatus for evaporation and/or the apparatus for evaporation and stripping preferably is in a range from 800 to 1200 mbar(abs), more preferred in a range from 900 to 1100 mbar(abs) and particularly in a range from 950 to 1050 mbar(abs).

Additionally or as an alternative it is also possible to remove the low boilers in the first stage by stripping. In this case it is preferred to preheat the diluted sulfuric acid to a temperature in the range from 30 to 180°C before feeding the diluted sulfuric acid into a stripping apparatus. For stripping, any stripping apparatus known to a skilled person can be used. A suitable stripping apparatuses for example is a stripping column which may contain externals, for example structured or random packing.

For stripping, the diluted sulfuric acid is brought into contact with a stripping gas, particularly steam. Preferably, the stripping gas is fed into the bottom of the stripping column and the diluted sulfuric acid at the top of the stripping column. Besides using an additional stripping gas, it is also possible to use a part of the vapor obtained by evaporating the diluted sulfuric acid as stripping gas. For this purpose, it is for example possible to evaporate the diluted sulfuric acid at the bottom of the column and to feed the diluted sulfuric acid at the top of the column. By this process, the vapor obtained by evaporation of the diluted sulfuric acid flows in countercurrent to the liquid diluted sulfuric acid which is fed at the top of the stripping column and low boilers are stripped by the vapor.

The apparatus used for evaporation and/or stripping can be any apparatus suitable for evaporation and/or stripping and known to a skilled person. Usually, such an apparatus for evaporation comprises a vessel with a heat exchange device inside or a vessel with an external heat exchanger and circulation loop. If an apparatus for evaporation and stripping is used, the apparatus comprises a stripping section which preferably is a column with internals, for example random or structured packing, and a heat exchange device at the bottom. The diluted acid is filled into the vessel, the circulation loop and/or the top of the stripping column. The non-evaporated liquid collects at the bottom of the vessel and/or stripping column and the vapors are removed by a vapor line which is connected to the top of the vessel and/or stripping column. The vapors removed from the apparatus used for evaporation and/or stripping are partly condensed and then emitted to the surroundings. However, as the vapors usually contain components which may be harmful to the environment, it is particularly preferred to feed the vapors to a gas purification unit before emitting the vapors into the surroundings. The gas purification unit can be any gas purification unit which is suitable for removing undesired components from the vapor and which is well known to a skilled person.

In the first stage a first concentrated sulfuric acid is obtained. The concentration of the first concentrated sulfuric acid is above the concentration of the diluted sulfuric acid below the concentration of the concentrated sulfuric acid obtained as product. Preferably, the concentration of the first sulfuric acid is in a range from 70 to 86 wt%, more preferred in a range from 72 to 84 wt% and particularly in a range from 76 to 81 wt%.

In one embodiment, the first concentrated sulfuric acid is fed into a second evaporation stage. The second evaporation stage preferably is operated under vacuum conditions at a temperature which does not allow the formation of solid carbonaceous precipitates and/or spontaneous decomposition of the organic impurities still contained in the first concentrated sulfuric acid. For establishing vacuum conditions, it is preferred to connect a vapor outlet of an evaporation unit used in the second evaporation stage with a vacuum unit, for example a vacuum pump. During operation of the second evaporation stage, vapors obtained in the evaporation unit of the second evaporation stage are partly condensed, for example by direct cooling in a counter-current flow column, particularly with sulfuric acid as cooling liquid, and/or by indirect cooling in a heat exchanger with a cooling medium, for example water. That part of the vapors which remains in gaseous form is exhausted to the vacuum unit and withdrawn from the process, preferably after passing a gas purification unit. In the vacuum unit the pressure for the second evaporation stage is set.

The gas purification unit may be the same as used for the vapors withdrawn from the first stage.

The pressure at which the second evaporation stage is operated preferably is in a range from 10 to 800 mbar(abs), more preferred in a range from 20 to 500 mbar(abs) and particularly in a range from 50 to 200 mbar(abs). The temperature of the second evaporation stage preferably is in a range from 120 to 200 °C, more preferred in a range from 140 to 190 °C and particularly in a range from 140 to 180 °C.

The second evaporation stage may be carried out in only one evaporation unit or in more than one evaporation unit which are connected in series. Preferably, the second evaporation stage is carried out in 1 to 4 evaporation units, more preferred in 1 to 3 evaporation units and particularly in 1 to 2 evaporation units. Particularly preferably, the second evaporation stage is carried out in one evaporation unit.

If the second evaporation stage is carried out in more than one evaporation unit which are connected in series, the temperature in the evaporation units increases from the first to the last evaporation unit being connected in series and/or the pressure decreases from the first to the last evaporation unit being connected in series, wherein each evaporation unit is operated within the conditions described above for the second evaporation unit.

In the second evaporation stage, particularly water is evaporated and the concentration of the sulfuric acid is increased. In the second evaporation stage a second concentrated sulfuric acid is obtained. The concentration of the second concentrated sulfuric acid is higher than the concentration of the first concentrated sulfuric acid and lower than the concentration of the concentrated sulfuric acid obtained as product of the process. Preferably, the concentration of the second concentrated sulfuric acid is in a range from 78 to 94 wt%, more preferred in a range from 80 to 92 wt% and particularly in a range from 84 to 89 wt%.

If the second evaporation stage is carried out, the second concentrated sulfuric acid or, if the second evaporation stage is not carried out, the first concentrated sulfuric acid is fed into the third evaporation stage. The third evaporation stage also preferably is carried out under vacuum conditions. However, in difference to the second evaporation stage, the third evaporation stage is carried out at a temperature at which the formation of solid carbonaceous precipitates and/or a spontaneous decomposition of the organic impurities contained in the first concentrated sulfuric acid or in the second concentrated sulfuric acid may occur.

The pressure at which the third evaporation stage is carried out is in a range from 10 to 800 mbar(abs), more preferred in a range from 20 to 500 mbar(abs) and particularly in a range from 50 to 200 mbar(abs) and at a temperature in a range from 180 to 250 °C, more preferred in a range from 190 to 250 °C and particularly in a range from 190 to 230 °C.

The third evaporation stage may comprise one evaporation unit or more than one evaporation units which are connected in series. Preferably, the third evaporation stage comprises 1 to 4 evaporation units, more preferred 1 to 3 evaporation units and particularly 1 to 2 evaporation units. Particularly preferably, the third evaporation stage is carried out in one evaporation unit.

The third evaporation stage preferably is operated in the same manner as the second evaporation stage. Particularly, vapors obtained in the evaporation unit of the third evaporation stage are partly condensed, for example by direct cooling in a counter-current flow column, particularly with sulfuric acid as cooling liquid, and/or by indirect cooling in a heat exchanger with a cooling medium, for example water. That part of the vapors which is not condensed and remains in the gaseous phase is passed to a vacuum unit, for example a vacuum pump, and removed from the process. As the vapors removed from the first and second evaporation stages, also the vapors removed from the third evaporation stage preferably passes a gas purification unit before being emitted into the environment.

It is possible to provide separate gas purification units for each evaporation stage or even for each evaporation unit. On the other hand it is also possible to combine the vapors of at least two evaporation units, more preferred to combine the vapors of at least two evaporation stages and particularly of all evaporation stages and feed the combined vapors into the gas purification unit.

The evaporation units used in the second evaporation stage, if carried out, and in the third evaporation stage may be any type of evaporator known to a skilled person. Suitable evaporators for example are falling film evaporators, circulation evaporators or horizontal evaporators. Particularly preferably, the evaporator used as evaporation unit in the second evaporation stage and in the third evaporation stage is a circulation evaporator and/or a horizontal evaporator.

Particularly for concentrating large amounts of diluted sulfuric acid it may be necessary to use more than one concentrating unit comprising the apparatuses for the first stage, the optional second evaporation stage and the third evaporation stage. In this case, depending on the amount of diluted sulfuric acid and the size of the apparatuses, at least two concentrating units are operated parallel, with all concentrating units preferably being designed and operated in the same way.

According to the invention, an oxidizing agent and/or a precursor of an oxidizing agent is fed into the third evaporation stage.

The oxidizing agent fed into the third evaporation stage may be any oxidizing agent which controllably reacts with the organic impurities for selectively decomposing the organic impurities forming volatile components. By this reaction it is avoided that solid carbonaceous precipitates are formed and deposit on equipment surfaces where they cause fouling. By this reaction it is further avoided that the organic impurities accumulate, for example in dead spaces or over a longer period, and that a spontaneous decomposition of the accumulated organic impurities which may result in equipment damage occurs. The volatile components which are produced by the reaction of the organic impurities with the oxidizing agent are removed from the third evaporation stage with the non-condensed vapors. The volatile components for example are nitrogen oxides, carbon monoxide and/or carbon dioxide. To avoid blowing the volatile components, particularly nitrogen oxides, into the environment, these are removed from the vapors in the gas purification unit.

Suitable oxidizing agents for the decomposition of nitroaromatic compounds as organic impurities are nitric acid, nitrous acid, nitrosylsulfonic acid and combination thereof. Particularly preferably, the oxidizing agent is nitric acid. If nitric acid is used as oxidizing agent, it is particularly preferred to take the nitric acid from the same storage container for nitric acid from which the nitric acid used for the nitration reaction is taken.

Besides feeding an oxidizing agent, it is also possible to feed a precursor of an oxidizing agent into the third evaporation stage. If a precursor of an oxidizing agent is fed into the third evaporation stage, the oxidizing agent is formed in situ in the third evaporation stage by chemical reaction of the precursor of the oxidizing agent. A suitable precursor of the oxidizing agent may be sodium nitrite. By adding sodium nitrite to the sulfuric acid, nitrous acid is generated which serves as oxidizing agent.

For an adequate cleaving of organic impurities, particularly the nitroaromatic compounds which still are contained in the first or second concentrated sulfuric acid which is fed into the third evaporation stage, the amount of oxidizing agent fed into the third evaporation stage and/or formed in situ in the third evaporation stage by conversion of the precursor of the oxidizing agent preferably is in a range from 0.01 to 100 g per kg concentrated sulfuric acid, more preferred in a range from 0.1 to 50 g per kg concentrated sulfuric acid and particularly in a range from 0.5 to 30 g per kg concentrated sulfuric acid. The amount of oxidizing agent per kg of concentrated sulfuric acid relates to the hypothetical pure oxidizing agent and not the amount of the aqueous solution.

To avoid diluting the sulfuric acid, it is further preferred if the oxidizing agent which is fed into the third evaporation stage has a concentration from 30 to 100 wt%, more preferred from 65 to 100 wt% and particularly from 85 to 100 wt%, each based on the amount of oxidizing agent and water. If a precursor of the oxidizing agent is fed into the third evaporation stage, the amount of the precursor is selected such that the amount of oxidizing agent generated in situ by chemical reaction of the precursor is in the above mentioned ranges.

For feeding the oxidizing agent or the precursor of the oxidizing agent into the third evaporation stage, it is possible to mix the first concentrated sulfuric acid, if the second evaporation stage is not carried out, or the second concentrated sulfuric acid, if the second evaporation stage is carried out, with the oxidizing agent and to feed the mixture of first or second concentrated sulfuric acid and oxidizing agent into the third evaporation stage.

Mixing of the first or second concentrated sulfuric acid and the oxidizing agent can be carried out for example by feeding the oxidizing agent or the precursor of the oxidizing agent into a feeding line through which the first or second concentrated sulfuric acid is fed into the third evaporation stage. Further, it is also possible to provide a mixing unit in which the first or second concentrated sulfuric acid and the oxidizing agent or the precursor of the oxidizing agent are mixed. The mixing unit can be any static mixer or dynamic mixer which allows mixing of two liquids.

Besides mixing the first or second concentrated sulfuric acid and the oxidizing agent or the precursor of the oxidizing agent, it is also possible to directly feed the oxidizing agent or the precursor of the oxidizing agent into the third evaporation stage. If the oxidizing agent or the precursor of the oxidizing agent is directly fed into the third evaporation stage, the oxidizing agent or precursor of the oxidizing agent can be fed into an evaporation unit of the third evaporation stage. However, particularly if a circulation evaporator is used as evaporation unit, it is preferred to feed the oxidizing agent or the precursor of the oxidizing agent into an evaporation loop. The evaporation loop for example can be a line between the vessel for vapor-liquid-separation and the heat exchanger or a line through which the liquid phase is circulated during the evaporation process.

The oxidizing agent or the precursor of the oxidizing agent may be added into the third evaporation stage continuously or discontinuously. However, it is preferred to feed the oxidizing agent or the precursor of the oxidizing agent continuously into the third evaporation stage.

For a controlled feeding of the oxidizing agent or precursor of the oxidizing agent into the third evaporation stage, it is particularly preferred to use a metering unit for feeding the oxidizing agent or precursor of the oxidizing agent into the third evaporation stage. The metering unit may be any metering unit for feeding a predetermined amount which is known to a skilled person. Particularly preferably, the metering unit is a mass control valve.

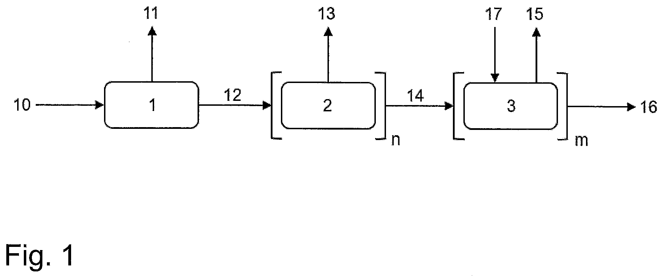

An illustrative embodiment of the invention is shown in the figure and explained in more detail in the following description.

The only figure shows a flow diagram of the inventive process for concentrating sulfuric acid.

For concentrating diluted sulfuric acid which may contain nitroaromatic compounds as organic impurities, the diluted sulfuric acid 10 is fed into a first stage 1. The first stage 1 preferably is an evaporation, a stripping section and/or a combination of evaporation and stripping section in which nitric acid and major parts of the organic impurities are stripped off from the diluted sulfuric acid 10. The nitric acid and the major parts of the organic impurities are removed as a gaseous exhaust stream 11.

In the first stage 1, a first concentrated sulfuric acid 12 is obtained which may be fed into a second evaporation stage 2. The second evaporation stage may comprise n evaporation units, with n being a number between 1 and 5. However, it is also possible to omit the second evaporation stage and feed the first concentrated sulfuric acid into a third evaporation stage 3.

In the second evaporation stage 2 the first concentrated sulfuric acid is further concentrated under vacuum conditions. By evaporation in the second evaporation stage, acidic vapors 13 are obtained which are partly condensed and the non-condensed vapors are exhausted to a vacuum unit. The second evaporation stage is operated at a temperature in the range from 120 to 200 °C, more preferred at a temperature in the range from 140 to 190 °C and particularly at a temperature in the range from 140 to 180 °C and a pressure in the range from 10 to 800 mbar(abs), more preferred in the range from 20 to 500 mbar(abs) and particularly in the range from 50 to 200 mbar(abs), wherein the temperature is below the limiting temperature at which a formation of solid carbonaceous precipitates or a spontaneous decomposition of the organic impurities takes place, even if the organic impurities are accumulated in equipment areas like dead bands and over a longer period.

In the second evaporation stage 2 a second concentrated sulfuric acid 14 is obtained which is fed into the third evaporation stage 3. In the third evaporation stage 3, the sulfuric acid is concentrated to a final concentration in the range from 80 to 99 wt%. Preferably, the concentrated sulfuric acid 16 which is obtained as product in the third evaporation stage 3 is recycled into a nitration process for producing nitroaromatic compounds, preferably nitrotoluene.

Like the second evaporation stage 2 also the third evaporation stage 3 is carried out under vacuum conditions, the acidic vapors 15 obtained by evaporation in the third evaporation stage are partly condensed and recycled into the third evaporation stage and the non-condensed vapors are exhausted to a vacuum unit.

The third evaporation stage 3 is operated at a temperature in the range from 180 to 250°C, more preferred at a temperature in the range from 190 to 250°C and particularly at a temperature in the range from 190 to 230°C and a pressure in the range from 10 to 800 mbar(abs), more preferred in the range from 20 to 500 mbar(abs) and particularly in the range from 50 to 200 mbar(abs). Due to the operating temperature above 180°C there is a risk of the formation of solid carbonaceous precipitates that deposit on equipment surfaces where they cause fouling. Due to the operating temperature above 180°C there is a further risk of spontaneous organic decomposition, particularly if the organic impurities are accumulated in equipment areas like dead bands and over a longer period. This spontaneous organic decomposition may cause pressure shocks and equipment damage.

To prevent the formation of solid carbonaceous precipitates or organic accumulation, an oxidizing agent 17 or a precursor of the oxidizing agent is fed into the third evaporation stage 3. Due to the oxidizing impact, the organic impurities which still are contained in the first or second concentrated sulfuric acid which is fed into the third evaporation stage 3, are selectively decomposed to volatile components before they start to form solid precipitates or to accumulate. These volatile components, for example nitrogen oxides, carbon monoxide and/or carbon dioxide are exhausted as part of the acidic vapors 15 to the vacuum unit.

The third evaporation stage may comprise 1 to 5 evaporation units. Suitable evaporation units for example are circulation evaporators, horizontal evaporators or falling film evaporators. Depending on the evaporator type and process design, the oxidizing agent 17 or the precursor of the oxidizing agent may be fed into an evaporation loop, for example on the suction side of the circulation pump, or into the first or second concentrated sulfuric acid 12, 14, before being fed into the third evaporation stage 3.

### Comparative example 1

Diluted sulfuric acid comprising 71 wt% sulfuric acid, 1 wt% nitric acid, 0.5 wt% dinitrotoluene and the remainder being water was fed into a first evaporation stage having a feed temperature of 135 °C. The first evaporation stage was operated at a pressure of 1 bar and a bottom temperature of 180 °C.

The thus obtained first concentrated sulfuric acid was withdrawn at the bottom of the first evaporation stage and fed into a second evaporation stage having a feed temperature of 180 °C. The second evaporation stage was operated at a pressure of 0.09 bar and a bottom temperature of 160 °C. At the bottom of the second evaporation stage a second concentrated sulfuric acid was obtained which then was fed into the third evaporation stage with a feed temperature of 160 °C. The third evaporation stage was operated at a pressure of 0.09 bar and a bottom temperature of 210 °C and the concentrated sulfuric acid obtained in the bottom of the third evaporation stage was withdrawn as product.

In this example, no oxidizing agent was added.

The TOC of the product was 0.023, the density of the product 1.833 g/ml and the concentration of the sulfuric acid 96 wt%. No solids were observed in the product and the product had a yellow color.

### Comparative example 2

Diluted sulfuric acid comprising 71 wt% sulfuric acid, 1 wt% nitric acid, 0.5 wt% dinitrotoluene and the remainder being water was fed into a first evaporation stage having a feed temperature of 135 °C. The first evaporation stage was operated at a pressure of 1 bar and a bottom temperature of 180 °C.

The thus obtained first concentrated sulfuric acid was withdrawn at the bottom of the first evaporation stage and fed into a second evaporation stage having a feed temperature of 180 °C. The second evaporation stage was operated at a pressure of 0.09 bar and a bottom temperature of 160 °C. At the bottom of the second evaporation stage a second concentrated sulfuric acid was obtained which then was fed into the third evaporation stage with a feed temperature of 160 °C. The third evaporation stage was operated at a pressure of 0.09 bar and a bottom temperature of 210 °C and the concentrated sulfuric acid obtained in the bottom of the third evaporation stage was withdrawn as product.

In this example, 5 g of 99.8% nitric acid per kg feed stream were added into the second stage as oxidizing agent.

The TOC of the product was 0.025, the density of the product 1.826 g/ml and the concentration of the sulfuric acid 95 wt%. No solids were observed in the product and the product had a yellow color.

### Example 1

Diluted sulfuric acid comprising 71 wt% sulfuric acid, 1 wt% nitric acid, 0.5 wt% dinitrotoluene and the remainder being water was fed into a first evaporation stage having a feed temperature of 135 °C. The first evaporation stage was operated at a pressure of 1 bar and a bottom temperature of 180 °C.

The thus obtained first concentrated sulfuric acid was withdrawn at the bottom of the first evaporation stage and fed into a second evaporation stage having a feed temperature of 180 °C. The second evaporation stage was operated at a pressure of 0.09 bar and a bottom temperature of 160 °C. At the bottom of the second evaporation stage a second concentrated sulfuric acid was obtained which then was fed into the third evaporation stage with a feed temperature of 160 °C. The third evaporation stage was operated at a pressure of 0.09 bar and a bottom temperature of 210 °C and the concentrated sulfuric acid obtained in the bottom of the third evaporation stage was withdrawn as product.

5 g of 99.8% nitric acid per kg feed stream were added into the third stage as oxidizing agent.

The TOC of the product was 0.015, the density of the product 1.837 g/ml and the concentration of the sulfuric acid 96 wt%. No solids were observed in the product and the product was colorless.

### Example 2

Diluted sulfuric acid comprising 71 wt% sulfuric acid, 1 wt% nitric acid, 0.5 wt% dinitrotoluene and the remainder being water was fed into a first evaporation stage having a feed temperature of 135 °C. The first evaporation stage was operated at a pressure of 1 bar and a bottom temperature of 180 °C.

The thus obtained first concentrated sulfuric acid was withdrawn at the bottom of the first evaporation stage and fed into a second evaporation stage having a feed temperature of 180 °C. The second evaporation stage was operated at a pressure of 0.09 bar and a bottom temperature of 160 °C. At the bottom of the second evaporation stage a second concentrated sulfuric acid was obtained which then was fed into the third evaporation stage with a feed temperature of 160 °C. The third evaporation stage was operated at a pressure of 0.09 bar and a bottom temperature of 180 °C and the concentrated sulfuric acid obtained in the bottom of the third evaporation stage was withdrawn as product.

In this example, 5 g of 99.8% nitric acid per kg feed stream were added into the third stage as oxidizing agent.

The TOC of the product was 0.042, the density of the product 1.823 g/ml and the concentration of the sulfuric acid 94 wt%. No solids were observed in the product and the product was colorless.

### Example 3

Diluted sulfuric acid comprising 71 wt% sulfuric acid, 1 wt% nitric acid, 0.5 wt% dinitrotoluene and the remainder being water was fed into a first evaporation stage having a feed temperature of 135 °C. The first evaporation stage was operated at a pressure of 1 bar and a bottom temperature of 180 °C.

The thus obtained first concentrated sulfuric acid was withdrawn at the bottom of the first evaporation stage and fed into the third evaporation stage having a feed temperature of 180 °C. The third evaporation stage was operated at a pressure of 0.09 bar and a bottom temperature of 210 °C and the concentrated sulfuric acid obtained in the bottom of the third evaporation stage was withdrawn as product.

In this example, 5 g of 99.8% nitric acid per kg of the feed stream were added into the third stage as oxidizing agent.

The TOC of the product was 0.024, the density of the product 1.826 g/ml and the concentration of the sulfuric acid 95 wt%. No solids were observed in the product and the product was colorless.

## Claims

1. A process for concentrating diluted sulfuric acid (10) which may comprise at least one nitroaromatic compound and/or nitric acid as impurities, comprising:
(a) feeding the diluted sulfuric acid (10) into a first stage (1) in which low boilers are removed by evaporation and/or stripping to obtain a first concentrated sulfuric acid (12);
(b) optionally feeding the first concentrated sulfuric acid (12) into a second evaporation stage (2) to obtain a second concentrated sulfuric acid (14);
(c) feeding the second concentrated sulfuric acid (14) into a third evaporation stage (3) if step (b) is carried out, or feeding the first concentrated sulfuric acid (12) into the third evaporation stage (3) if step (b) is not carried out, to obtain concentrated sulfuric acid (16) as product,
wherein the third evaporation stage (3) is operated at a pressure in the range from 10 to 800 mbar (abs) and a temperature in the range from 180 to 250°C and wherein an oxidizing agent (17) and/or a precursor of an oxidizing agent is fed into the third evaporation stage (3).

2. The process according to claim 1, wherein at least one of the at least one nitroaromatic compound is mononitrotoluene and/or dinitrotoluene.

3. The process according to claim 1 or 2, wherein the oxidizing agent is selected from the group consisting of nitric acid, nitrous acid, nitrosylsulfonic acid and combinations thereof, and/or the precursor of the oxidizing agent is sodium nitrite.

4. The process according to any of claims 1 to 3, wherein the amount of oxidizing agent is in the range from 0.01 to 100 g per kg concentrated sulfuric acid.

5. The process according to any of claims 1 to 4, wherein the first stage (1) comprises an evaporation which is operated at a temperature in the range from 130 to 200 °C and a pressure in the range from 800 to 1200 mbar(abs).

6. The process according to any of claims 1 to 5, wherein the second evaporation stage (2) is operated at a pressure in the range from 10 to 800 mbar (abs) and a temperature in the range from 120 to 200 °C.

7. The process according to any of claims 1 to 6, wherein the second evaporation stage (2), if carried out, comprises 1 to 5 evaporation units.

8. The process according to any of claims 1 to 7, wherein the third evaporation stage (3) comprises 1 to 5 evaporation units.

9. The process according to any of claims 1 to 8, wherein the evaporation units used in the third evaporation stage (3) are circulation evaporators, horizontal evaporators or falling film evaporators.

10. The process according to any of claims 1 to 9, wherein the oxidizing agent and/or the precursor of the oxidizing agent is fed into an evaporation loop.

11. The process according to any of claims 1 to 9, wherein the oxidizing agent and/or the precursor of the oxidizing agent is mixed with the second concentrated sulfuric acid (14) if step (b) is carried out, or with the first concentrated sulfuric acid (12) if step (b) is not carried out before feeding into the third evaporation stage (3).

12. The process according to any of claims 1 to 11, wherein the diluted sulfuric acid (10) has a concentration in the range from 60 to 85 wt%.

13. The process according to any of claims 1 to 12, wherein the concentrated sulfuric acid (16) has a concentration in the range from 80 to 99 wt%.

14. The process according to any of claims 1 to 13, wherein the diluted sulfuric acid (10) is obtained in a process for producing nitrotoluene and wherein the concentrated sulfuric acid (16) preferably is recycled into the process for producing nitrotoluene.

## Patentansprüche

1. Verfahren zum Konzentrieren von verdünnter Schwefelsäure (10), die mindestens eine nitroaromatische Verbindung und/oder Salpetersäure als Verunreinigungen umfassen kann, umfassend:
(a) das Einspeisen der verdünnten Schwefelsäure (10) in eine erste Stufe (1), in der Leichtsieder durch Verdampfen und/oder Strippen entfernt werden, unter Erhalt einer ersten konzentrierten Schwefelsäure (12),
(b) gegebenenfalls das Einspeisen der ersten konzentrierten Schwefelsäure (12) in eine zweite Verdampfungsstufe (2), unter Erhalt einer zweiten konzentrierten Schwefelsäure (14),
(c) das Einspeisen der zweiten konzentrierten Schwefelsäure (14) in eine dritte Verdampfungsstufe (3), wenn Schritt (b) durchgeführt wird, oder das Einspeisen der ersten konzentrierten Schwefelsäure (12) in die dritte Verdampfungsstufe (3), wenn Schritt (b) nicht durchgeführt wird, unter Erhalt der konzentrierten Schwefelsäure (16) als Produkt,
wobei die dritte Verdampfungsstufe (3) bei einem Druck im Bereich von 10 bis 800 mbar (abs) und einer Temperatur im Bereich von 180 bis 250°C betrieben wird und wobei ein Oxidationsmittel (17) und/oder ein Vorläufer eines Oxidationsmittels in die dritte Verdampfungsstufe (3) eingespeist wird.

2. Verfahren nach Anspruch 1, wobei mindestens eine der mindestens einen nitroaromatischen Verbindung Mononitrotoluol und/oder Dinitrotoluol ist.

3. Verfahren nach Anspruch 1 oder 2, wobei das Oxidationsmittel aus der aus Salpetersäure, salpetriger Säure, Nitrosylsulfonsäure und Kombinationen davon bestehenden Gruppe ausgewählt ist und/oder der Vorläufer des Oxidationsmittels Natriumnitrit ist.

4. Verfahren nach einem der Ansprüche 1 bis 3, wobei die Menge an Oxidationsmittel im Bereich von 0,01 bis 100 g pro kg konzentrierter Schwefelsäure liegt.

5. Verfahren nach einem der Ansprüche 1 bis 4, wobei die erste Stufe (1) eine Verdampfung umfasst, die bei einer Temperatur im Bereich von 130 bis 200 °C und einem Druck im Bereich von 800 bis 1200 mbar (abs) betrieben wird.

6. Verfahren nach einem der Ansprüche 1 bis 5, wobei die zweite Verdampfungsstufe (2) bei einem Druck im Bereich von 10 bis 800 mbar (abs) und einer Temperatur im Bereich von 120 bis 200 °C betrieben wird.

7. Verfahren nach einem der Ansprüche 1 bis 6, wobei die zweite Verdampfungsstufe (2), falls durchgeführt, 1 bis 5 Verdampfungseinheiten umfasst.

8. Verfahren nach einem der Ansprüche 1 bis 7, wobei die dritte Verdampfungsstufe (3) 1 bis 5 Verdampfungseinheiten umfasst.

9. Verfahren nach einem der Ansprüche 1 bis 8, wobei als Verdampfungseinheiten in der dritten Verdampfungsstufe (3) Kreislaufverdampfer, Horizontalverdampfer oder Fallfilmverdampfer eingesetzt werden.

10. Verfahren nach einem der Ansprüche 1 bis 9, wobei das Oxidationsmittel und/oder der Vorläufer des Oxidationsmittels in eine Verdampfungsschleife eingespeist wird.

11. Verfahren nach einem der Ansprüche 1 bis 9, wobei das Oxidationsmittel und/oder der Vorläufer des Oxidationsmittels mit der zweiten konzentrierten Schwefelsäure (14) gemischt wird, wenn Schritt (b) durchgeführt wird, oder mit der ersten konzentrierten Schwefelsäure (12) gemischt wird, wenn Schritt (b) nicht vor der Einspeisung in die dritte Verdampfungsstufe (3) durchgeführt wird.

12. Verfahren nach einem der Ansprüche 1 bis 11, wobei die verdünnte Schwefelsäure (10) eine Konzentration im Bereich von 60 bis 85 Gew.-% aufweist.

13. Verfahren nach einem der Ansprüche 1 bis 12, wobei die konzentrierte Schwefelsäure (16) eine Konzentration im Bereich von 80 bis 99 Gew.-% aufweist.

14. Verfahren nach einem der Ansprüche 1 bis 13, wobei die verdünnte Schwefelsäure (10) in einem Verfahren zur Herstellung von Nitrotoluol erhalten wird und wobei die konzentrierte Schwefelsäure (16) vorzugsweise in das Verfahren zur Herstellung von Nitrotoluol zurückgeführt wird.

## Revendications

1. Procédé de concentration d'acide sulfurique dilué (10) pouvant comprendre au moins un composé nitroaromatique et/ou de l'acide nitrique comme impuretés, comprenant :
(a) l'introduction de l'acide sulfurique dilué (10) dans une première étape (1) dans laquelle des composés à bas point d'ébullition sont éliminés par évaporation et/ou extraction pour obtenir un premier acide sulfurique concentré (12) ;
(b) optionnellement l'introduction du premier acide sulfurique concentré (12) dans une deuxième étape d'évaporation (2) pour obtenir un deuxième acide sulfurique concentré (14) ;
(c) l'introduction du deuxième acide sulfurique concentré (14) dans une troisième étape d'évaporation (3) si l'étape (b) est effectuée, ou l'introduction du premier acide sulfurique concentré (12) dans la troisième étape d'évaporation (3) si l'étape (b) n'est pas effectuée, pour obtenir de l'acide sulfurique concentré (16) comme produit,
la troisième étape d'évaporation (3) étant mise en œuvre à une pression dans la plage de 10 à 800 mbar (abs) et à une température dans la plage de 180 à 250°°C, et un agent oxydant (17) et/ou un précurseur d'un agent oxydant étant introduits dans la troisième étape d'évaporation (3).

2. Procédé selon la revendication 1, dans lequel au moins un de l'au moins un composé nitroaromatique est le mononitrotoluène et/ou le dinitrotoluène.

3. Procédé selon la revendication 1 ou 2, dans lequel l'agent oxydant est choisi dans le groupe constitué de l'acide nitrique, de l'acide nitreux, de l'acide nitrosylsulfonique et de leurs combinaisons, et/ou le précurseur de l'agent oxydant est le nitrite de sodium.

4. Procédé selon l'une quelconque des revendications 1 à 3, dans lequel la quantité d'agent oxydant est dans la plage de 0,01 à 100 g par kg d'acide sulfurique concentré.

5. Procédé selon l'une quelconque des revendications 1 à 4, dans lequel la première étape (1) comprend une évaporation qui est mise en œuvre à une température dans la plage de 130 à 200 °C et à une pression dans la plage de 800 à 1200 mbar(abs).

6. Procédé selon l'une quelconque des revendications 1 à 5, dans lequel la deuxième étape d'évaporation (2) est mise en œuvre à une pression dans la plage de 10 à 800 mbar (abs) et à une température dans la plage de 120 à 200 °C.

7. Procédé selon l'une des revendications 1 à 6, **caractérisé en ce que** la deuxième étape d'évaporation (2), si elle est réalisée, comprend de 1 à 5 unités d'évaporation.

8. Procédé selon l'une quelconque des revendications 1 à 7, dans lequel la troisième étape d'évaporation (3) comprend de 1 à 5 unités d'évaporation.

9. Procédé selon l'une des revendications 1 à 8, **caractérisé en ce que** les unités d'évaporation utilisées dans la troisième étape d'évaporation (3) sont des évaporateurs à circulation, des évaporateurs horizontaux ou des évaporateurs à film tombant.

10. Procédé selon l'une quelconque des revendications 1 à 9, dans lequel l'agent oxydant et/ou le précurseur de l'agent oxydant sont introduits dans une boucle d'évaporation.

11. Procédé selon l'une quelconque des revendications 1 à 9, dans lequel l'agent oxydant et/ou le précurseur de l'agent oxydant sont mélangés avec le deuxième acide sulfurique concentré (14) si l'étape (b) est effectuée, ou avec le premier acide sulfurique concentré (12) si l'étape (b) n'est pas effectuée avant l'introduction dans la troisième étape d'évaporation (3).

12. Procédé selon l'une quelconque des revendications 1 à 11, dans lequel l'acide sulfurique dilué (10) a une concentration dans la plage de 60 à 85 % en poids.

13. Procédé selon l'une quelconque des revendications 1 à 12, dans lequel l'acide sulfurique concentré (16) a une concentration dans la plage de 80 à 99 % en poids.

14. Procédé selon l'une quelconque des revendications 1 à 13, dans lequel l'acide sulfurique dilué (10) est obtenu dans un procédé de production de nitrotoluène et dans lequel l'acide sulfurique concentré (16) est de préférence recyclé dans le procédé de production de nitrotoluène.
